# FASCICULE DE BREVET EUROPEEN

(11) **EP 2 215 446 B1**
(45) Date de publication et mention de la délivrance du brevet: **13.04.2011**
(21) Numéro de dépôt: 08854981.1
(22) Date de dépôt: 03.11.2008
(51) Int. Cl.: G01N 1/00, B01F 3/02, G01N 33/00

(54) **PROCÉDÉ ET DISPOSITIF DE DÉLIVRANCE DE MÉLANGES DE GAZ POUR UN ANALYSEUR**
VERFAHREN UND VORRICHTUNG ZUR ZUFUHR VON GASMISCHUNGEN IN EIN ANALYSEGERÄT
METHOD AND DEVICE FOR SUPPLYING GAS MIXTURES FOR AN ANALYSER

(30) Priorité: 27.11.2007 FR 0759342
(43) Date de publication de la demande: 11.08.2010
(73) Titulaire: L'Air Liquide Société Anonyme pour l'Etude et l'Exploitation des Procédés Georges Claude, 75007 Paris (FR)
(72) Inventeur: BOSSOUTROT, Valérie, F-78940 La Queue les Yvelines (FR); KOPPEL, Joerg, 71640 Ludwigsburg (DE); LEPIC, Séverine, F-75008 Paris (FR); PAOLI, Hervé, F-92500 Rueil Malmaison (FR)
(74) Mandataire: Conan, Philippe Claude
(86) Numéro de dépôt international: PCT/FR2008/051977
(87) Numéro de publication internationale: WO 2009/068767

(56) Documents cités:
- DE-C1- 19 842 413
- US-A- 5 214 952
- US-A1- 2001 032 668

## Description

La présente invention concerne un dispositif d'analyse d'un fluide ainsi qu'un procédé de délivrance d'au moins un mélange de gaz vers notamment un analyseur de fluides.

Les fluides analysables peuvent être soit gazeux, soit liquides. Ils peuvent être obtenus par prélèvement direct sur un procédé industriel à des visées de contrôle qualité ou par prélèvement dans une atmosphère donnée par exemple en air ambiant à des fins de surveillance ou de contrôle.

Les analyseurs utilisés pour mesurer de faibles concentrations d'espèces chimiques dans un échantillon de fluide sont sensibles aux caractéristiques des gaz purs et mélanges de gaz qui les alimentent. Les gaz purs ou mélanges de gaz sont mis en oeuvre pour conduire l'échantillon jusqu'au détecteur et pour réaliser un point « zéro » lors de l'étalonnage, indispensable dans le domaine des analyses de fluides. Ils peuvent également servir au fonctionnement du détecteur lui-même. Ces gaz purs ou mélanges de gaz sont appelés gaz d'instrumentation. Ces gaz d'instrumentation sont par exemple, l'hélium, l'azote, l'air, ou des mélanges tels que H₂/He, H₂/Ar, Char, CO₂/Ar, H₂/N₂.

La teneur en impuretés présentes dans ces gaz d'instrumentation et les spécifications de réalisation de ceux-ci sont des paramètres qui influent sur la sensibilité des analyseurs et la reproductibilité des analyses.

Les niveaux et la nature des impuretés contenues dans les gaz d'instrumentation permettent d'atteindre une qualité équivalente à une pureté de 99,999%. Les impuretés les plus fréquemment garanties sont l'humidité et les hydrocarbures. Les oxydes de carbone (CO, CO₂) et l'oxygène peuvent également faire l'objet de garanties.

Du fait des réglementations toujours plus contraignantes, les laboratoires d'analyse doivent mesurer des concentrations de plus en plus faibles. L'amélioration de la performance des analyseurs est par conséquent focalisée sur leur limite de détection et leur précision. Les garanties aujourd'hui offertes pour les gaz d'instrumentation ne suffisent plus à satisfaire ces exigences aussi bien en termes de pureté que de précision de réalisation. En effet, les impuretés présentes en concentration trop importante vont perturber le bruit de fond des analyseurs dégradant ainsi leur sensibilité. La gamme des impuretés garanties peut être insuffisante et devenir une source d'interférences sur les mesures. Par exemple les impuretés garanties sont l'humidité et l'oxygène, alors que celles qui sont critiques pour l'analyse sont les hydrocarbures, une spécification supplémentaire permettra de limiter les interférences sur les analyses. Enfin, la différence entre les écarts de compositions, d'un mélange à un autre, trop élevée par rapport à la réalisation théoriquement prévue des proportions des différents composants des gaz d'instrumentation sont une source d'imprécision sur les résultats analytiques.

L'exemple particulier du détecteur à ionisation de flamme (FID) comme analyseur consiste en une flamme alimentée par un mélange hydrogène/hélium et par de l'air et une plaque collectrice. L'échantillon à analyser passe à travers une flamme qui décompose les molécules organiques et produit des ions. Ceux-ci sont récupérés sur une électrode polarisée et produisent ainsi un signal électrique. Le FID est extrêmement sensible et offre une large gamme dynamique. Les FID sont utilisés pour la détection d'hydrocarbures comme par exemple le méthane, l'éthane ou encore l'acétylène. L'échantillon à analyser est mélangé au préalable avec le mélange d'instrumentation combustible dans une zone préchauffée. Les ions et les électrons formés dans la flamme sont collectés et permettent ainsi à un courant de circuler dans un circuit externe. Le courant est proportionnel au taux d'ions qui dépend de la concentration en hydrocarbures des fluides à analyser. Le courant est détecté par un électromètre approprié et affiché sur une sortie analogique. Ainsi, le FID offre une lecture rapide, précise (jusqu'au ppb) et continue de la concentration en hydrocarbures.

Le FID est alimenté par deux mélanges de gaz : Hydrogène / Hélium (H₂/He) dans des proportions respectives bien définies, par exemple de 40% et 60 % et Oxygène / Azote (O₂/N₂) dans des proportions respectives bien définies, par exemple de 20% et 80 %. La variation des écarts par rapport à la réalisation théoriquement prévue des proportions des différents composants d'un mélange à un autre constitue une source d'incertitudes sur les résultats des analyses réalisées à l'aide de FID. Pour améliorer la fiabilité des analyses réalisées par FID, les mélanges H₂/He et O₂/N₂, dits gaz de flamme, doivent donc présenter des précisions de réalisation stables d'un mélange à un autre permettant ainsi de limiter l'influence de ce paramètre sur les mesures. En outre le contenu en impuretés présentes dans les gaz d'instrumentation est également un facteur à prendre en considération et ce d'autant plus que des faibles teneurs (inférieures ou égales à la partie par million) doivent être analysées par FID.

Ces spécifications doivent donc être améliorées pour assurer la fiabilité des analyses.

Par exemple pour les FID, les gaz de flamme sont aujourd'hui fournis en bouteilles avec des précisions de réalisation comprises entre 1 et 2 % absolus pour le contenu en Hydrogène dans le cas du mélange H₂/He et entre 0.5 et 1% absolus pour le contenu en Oxygène dans la cas du mélanges O₂/N₂. La variation de la composition de ces mélanges d'une bouteille à une autre est une source d'erreur pour le client final. En effet, une variation de 2 % absolus sur la concentration en H₂ dans le mélange H₂/He peut générer jusqu'à 30 % de variation du signal FID obtenu pour l'analyse d'hydrocarbures. De même une variation de 2 % du contenu en oxygène dans le mélange O₂/N₂ alimentant le FID, génère une variation de 10 % du signal FID.

En outre le contenu en impuretés est un paramètre critique pour la fiabilité des analyses. Une concentration en impuretés de 40 ppb génère une hausse du signal de 23% lorsqu'un échantillon d'air zéro est analysé par FID Total. Or à ce jour le niveau en impuretés usuellement garanti dans un mélange préparé en bouteille est compris entre 50 et 100 ppbv. Par exemple pour les FID, la fourniture en bouteilles de mélanges H₂/He et O₂/N₂ pour alimenter les FID présente donc l'inconvénient de générer des incertitudes importantes sur les résultats d'analyses qui seront d'autant plus critiques que les teneurs à analyser seront faibles. La fourniture de tels mélanges en bouteille ne garantit donc pas une reproductibilité dans le temps, ni de leur composition, ni de leur pureté.

Cette garantie correspond à un mode de préparation des emballages et de remplissage des mélanges qui permet de réaliser un grand nombre de produits à des coûts économiquement viables. L'évolution des besoins en termes de précision et de niveaux d'impuretés garantis implique que la réalisation de ces mélanges en bouteille nécessiterait une modification des procédés et des installations existants, ce qui entrainerait une augmentation significative des coûts de production. Ainsi, il semble que pour ce types de mélanges, les limites des précisions de réalisation par les centres de conditionnement soient aujourd'hui atteintes ; tout du moins pour fabriquer un grand nombre de ces mélanges. Quant aux impuretés, les niveaux garantis fluctuent selon les sources utilisées, le procédé de préparation des bouteilles de gaz et le procédé de remplissage de ces bouteilles.

On connaît le document US-A-5214952 qui décrit un appareil de calibration utilisant des moyens de purification des gaz situés en amont de l'endroit où se mélangent les gaz.
Le document US 2001/032668 A1 décrit aussi un système dans lequel la filtration des gaz a lieu en amont de leur mélange.
Il en va de même pour le système décrit dans le document DE 198 42 413 C1.

Un but de la présente invention est de pallier tout ou partie des inconvénients relevés ci-dessus.

A cette fin, l'invention, consiste en un dispositif d'analyse d'un fluide comprenant :
- un analyseur, et
- un système d'alimentation en gaz d'instrumentation dudit analyseur;
   caractérisé en ce que le système d'alimentation comprend au moins un mélangeur générant ledit gaz d'instrumentation et au moins un épurateur situé en aval dudit au moins un mélangeur et en amont de l'analyseur.

Selon un mode de réalisation de l'invention, le système d'alimentation en gaz d'instrumentation dudit analyseur comprend en outre au moins un épurateur situé en amont d'au moins un mélangeur.

L'avantage d'avoir un seul épurateur en aval du mélangeur est de simplifier l'ensemble du procédé de délivrance. En revanche, l'ajout d'un épurateur en amont du mélangeur permet de retirer des impuretés spécifiques d'au moins un des gaz purs en entrée que l'on ne pourrait pas retirer du mélange final en raison de contraintes techniques et/ou de sécurité.

Par ailleurs, des modes de réalisation de l'invention peuvent comporter l'une ou plusieurs des caractéristiques suivantes :
- L'analyseur est un détecteur à ionisation de flamme. L'analyseur peut également être choisi parmi la liste : catharomètre (TCD), détecteur à capture d'électrons (ECD), détecteur à photo-ionisation, détecteur à chimiluminescence, détecteur électrochimique, détecteur à ionisation d'hélium, détecteur à décharge ionisante, détecteur à émission de plasma, détecteur à émission atomique, analyseur de gaz réducteur.
- Par exemple un détecteur à ionisation de flamme est alimenté par deux mélanges de gaz d'instrumentation, l'un étant un mélange Hydrogène/Hélium, l'autre un mélange Oxygène/Azote ; le système d'alimentation étant constitué d'un premier épurateur situé en aval d'un premier mélangeur générant le mélange Hydrogène/Hélium et en amont de l'analyseur et d'un deuxième épurateur situé en aval d'un deuxième mélangeur générant le mélange Oxygène/Azote et en amont de l'analyseur.
- En particulier, lorsque l'analyseur à alimenter est un détecteur à ionisation de flamme, le mélange Hydrogène/Hélium présente des proportions respectives de 35% à 45% en Hydrogène, de préférence 40% et de 55% à 65% en Hélium, de préférence 60% ; le mélange Oxygène/Azote présente des proportions respectives de 15% à 25% en Oxygène, de préférence 20% et de 75% à 85% en Azote, de préférence 80 %.
- Ledit au moins un mélangeur comprend un composant choisi parmi un régulateur de débits massiques, des orifices soniques, des barillets d'orifices calibrés de différents diamètres ou des vannes de régulation. Le paramétrage de seuils prédéfinis pour les régulateurs de débits massiques ou l'utilisation de barillets d'orifices calibrés de différents diamètres permettent de générer des mélanges de concentrations différentes et/ou de faire varier le débit total. Des composants tels que des régulateurs ou des capteurs de pression et vannes pneumatiques peuvent être intégrés dans ledit au moins un mélangeur, pour assurer un fonctionnement optimal des organes de régulation du débit.
- Ledit au moins un épurateur peut être, selon le gaz d'entré et le type d'impureté à éliminer, composé d'un ou plusieurs des éléments choisis parmi les éléments suivants: un filtre à particules, un catalyseur comprenant des métaux nobles et/ou des oxydes métalliques, un piège cryogénique, un ou plusieurs adsorbants éventuellement répartis en plusieurs lits successifs, comme par exemple, le charbon actif, l'alumine activée, ou différents types de zéolithe.

Selon un mode de réalisation de l'invention, ledit au moins un épurateur peut également comporter une alarme ou un avertissement tel qu'un signal sonore, visuel, ou autre message signalant que l'épurateur a atteint la fin de la durée de vie garantie. Un tel dispositif permet d'informer l'utilisateur pour qu'il procède soit au changement de l'épurateur soit au basculement sur une alimentation de secours lui offrant les mêmes spécifications. En variante, cette alarme peut être couplée à un arrêt automatique du mélangeur pour éviter que les analyses ne soient erronées à cause de la dégradation des performances de l'épurateur.

L'alimentation de secours peut être constituée soit d'un gaz en bouteille de composition connue, soit d'une dérivation vers un autre épurateur.

En variante supplémentaire, l'épuration est assurée par un moyen d'épuration qui s'auto régénère. Un tel moyen d'épuration est par exemple un PSA (en anglais, Pressure Swing Adsorption) ou un TSA (en anglais, Temperature Swing Adsorption). Dans ces deux derniers exemples, deux capacités sont placées en parallèle sur le passage du gaz et remplies par un adsorbant (tel que le charbon actif, alumine activé, zéolithe). Lorsque la première permet le passage du gaz vers le mélangeur, la seconde est en régénération. Cette régénération peut se faire au moyen d'une variation de pression (PSA) ou d'une augmentation de la température sous flux de gaz (TSA).

Selon un mode de réalisation de l'invention, un régulateur de pression est situé en aval dudit au moins un épurateur.

L'invention a aussi pour objet un système d'alimentation en gaz d'instrumentation d'un analyseur de fluide, constitué d'au moins un mélangeur générant ledit gaz d'instrumentation associé à au moins un épurateur situé en aval dudit au moins un mélangeur. Ledit système d'alimentation étant intégré dans un même dispositif d'analyse de fluide.

L'invention a aussi pour objet un système d'alimentation en gaz d'instrumentation d'un détecteur à ionisation de flamme, constitué de deux mélangeurs générant chacun un mélange de gaz d'instrumentation, chaque mélangeur étant situé en amont d'un épurateur.

L'invention a également pour objet l'utilisation d'un système d'alimentation tel que décrit précédemment, pour la délivrance d'au moins un mélange de gaz d'instrumentation vers un analyseur de fluide.

L'invention a encore pour objet un procédé de délivrance d'au moins un mélange de gaz vers un analyseur de fluide comprenant les étapes :
a) mélange d'au moins deux gaz purs mettant en oeuvre un mélangeur,
b) épuration du mélange obtenu à l'étape a) au moyen d'un épurateur générant un mélange de gaz d'instrumentation pour ledit analyseur ;
   caractérisé par le fait que ces étapes se déroulent sur un même site au moyen d'un unique équipement.

Un tel procédé peut également comprendre une étape, d'épuration d'au moins un gaz à mélanger, antérieure à l'étape a).

L'invention a encore pour objet un procédé de délivrance de deux mélanges de gaz vers un détecteur à ionisation de flamme comprenant les étapes :
a) formation d'un premier mélange constitué de 40% d'hydrogène et de 60% d'hélium mettant en oeuvre un premier mélangeur et formation d'un deuxième mélange constitué de 20% d'oxygène et de 80% d'azote mettant en oeuvre un deuxième mélangeur,
b) épuration du premier mélange obtenu à l'étape a) au moyen d'un premier épurateur générant un premier mélange de gaz d'instrumentation pour le détecteur à ionisation de flamme et épuration du deuxième mélange obtenu à l'étape a) au moyen d'un deuxième épurateur générant un deuxième mélange de gaz d'instrumentation pour ledit détecteur à ionisation de flamme ;
   caractérisé par le fait que ces étapes se déroulent sur un même site au moyen d'un unique équipement.

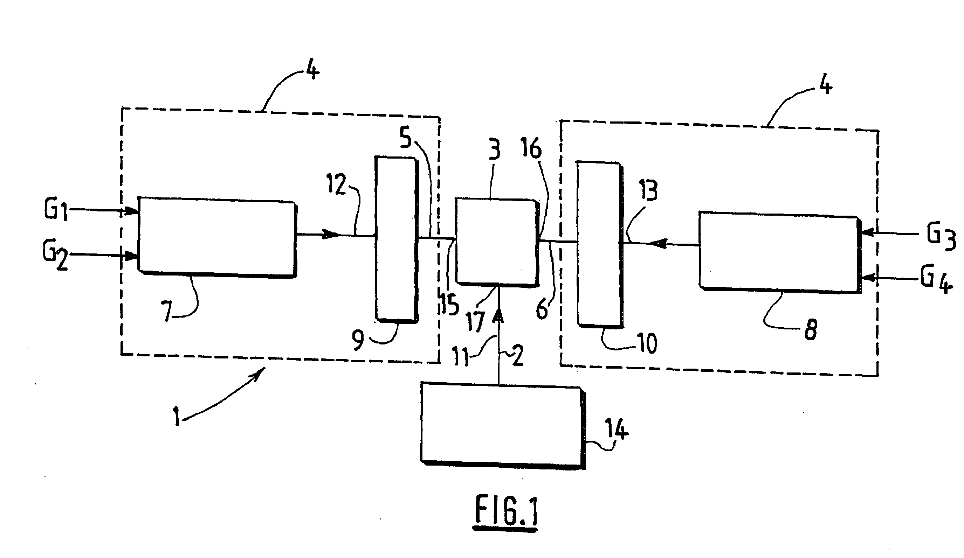

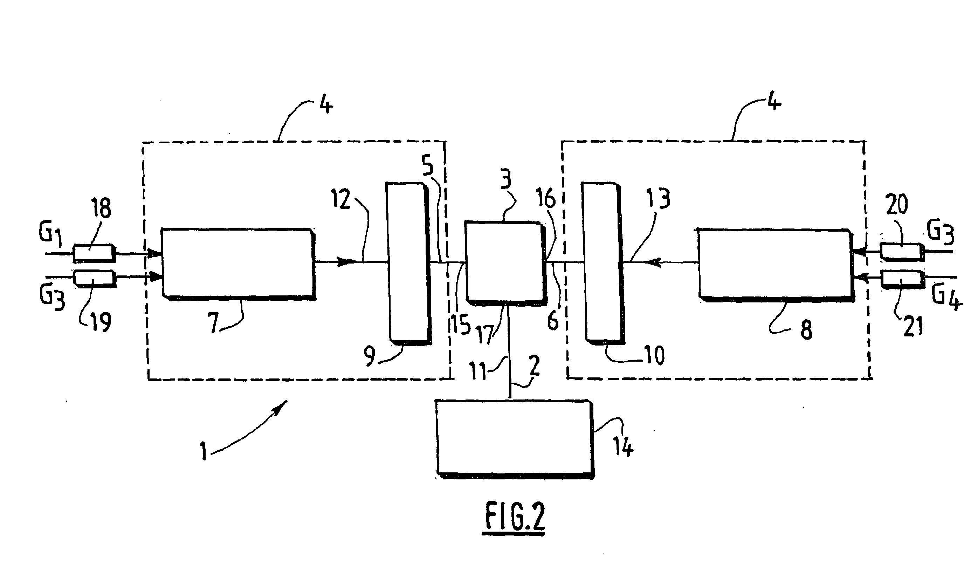

D'autres particularités et avantages apparaîtront à la lecture de la description ci-après, faite en référence aux figures 1 et 2.
- La figure 1 représente un schéma d'un dispositif d'analyse d'un fluide selon l'invention.
- La figure 2 représente un schéma d'une variante d'un dispositif d'analyse d'un fluide selon l'invention.

Sur la figure 1 est représenté un dispositif 1 d'analyse d'un fluide 2 contenu dans un réservoir 14. Le fluide 2 est par exemple un prélèvement d'air ambiant ou un échantillon de gaz d'échappement. Le dispositif 1 est constitué d'un équipement 4 ou système d'alimentation en gaz d'instrumentation d'un analyseur 3. L'équipement 4 est ici constitué de deux parties identiques, chacune constituée d'un épurateur 9 ou 10 situé en aval d'un mélangeur 7 ou 8. Les gaz G1 à G4 en entrée sont des gaz purs comme par exemple Hélium, Azote, Hydrogène, Argon, Méthane, ou Dioxyde de Carbone. Ces gaz purs sont notamment délivrés sous forme de bouteilles ou bien à partir de générateurs. Leurs spécifications en termes d'impuretés sont compatibles avec les capacités d'épuration de l'épurateur 9 ou 10 placé en amont de l'analyseur 7 ou 8.

Les technologies existantes pour le mélangeur 7 ou 8 permettent de générer des mélanges 5 ou 6 avec une incertitude inférieure ou égale à 0.5 % absolus sur la concentration du composant minoritaire. Il peut s'agir de régulateurs de débits massiques ou des orifices soniques ou des vannes de régulation.

L'épurateur 9 ou 10 permet de réduire le contenu en impuretés critiques pour l'analyse. Par exemple l'épurateur peut être un catalyseur composé d'oxydes métalliques pour convertir les hydrocarbures en CO₂ et en H₂O. Il peut être associé à des adsorbants permettant de piéger ces impuretés pour limiter l'impact sur la mesure.

L'ensemble de cet équipement de délivrance de mélange de gaz permet de générer des mélanges précis ayant des teneurs en impuretés faibles. L'objet de la présente invention, qui constitue une alternative aux mélanges en bouteilles, est un moyen de réduire la contribution des mélanges d'alimentation des analyseurs à l'incertitude analytique.

Les gaz purs comme G1 et G2, (ou G3 et G4) entrent dans le mélangeur 7 (respectivement 8), ce dernier fournissant un mélange G1/G2 circulant dans une conduite 12 (respectivement G3/G4 circulant dans une conduite 13). L'épurateur 9 (respectivement 10) permet de réduire le contenu en impuretés critiques du mélange G1/G2 (respectivement G3/G4) et ainsi fournir un mélange 5 (respectivement 6). Les mélanges 5 et 6 représentent chacun un gaz d'instrumentation pour l'analyse du fluide 2 issu du réservoir 14. Ledit fluide 2 circule dans une conduite 11 vers l'analyseur 3, vers lequel circulent aussi les gaz d'instrumentation. Selon un mode particulier de l'invention, l'équipement 4 est composé d'une seule section. Une section est constituée d'un mélangeur 7, d'un épurateur 9 et d'une conduite 12 dans laquelle circule le gaz d'instrumentation 5. Un dispositif selon l'invention peut aussi comporter un équipement 4 constitué de plus de deux sections.

Si l'analyseur 3 est un détecteur à ionisation de flamme, l'équipement 4 ou système d'alimentation en gaz d'instrumentation comprend un premier épurateur 9 situé en aval d'un premier mélangeur 7, générant un mélange 5 Hydrogène (G1) / Hélium (G2) circulant dans la conduite 12. Un deuxième épurateur 10 est situé en aval d'un deuxième mélangeur 8, générant un mélange 6 Oxygène (G3) / Azote (G4) circulant dans la conduite 13. Cet épurateur 10 est situé en amont de l'analyseur 3 destiné à analyser le fluide 2 issu de l'échantillon 14, circulant dans la conduite 11 et se dirigeant vers l'analyseur 3 par l'entrée 17.

Les mélanges 5 et 6 alimentent l'analyseur 3 via les entrées 15 et 16 et conduisent le fluide 2 de l'échantillon 14 dans la partie de l'analyseur 3 où seront réalisées les mesures. Ces gaz de flamme 5 et 6 servent également à réaliser un point « zéro » lors de l'étalonnage de l'analyseur 3.

Sur la figure 2 est représenté un dispositif 1 d'analyse d'un fluide 2 contenu dans un réservoir 14. A la différence du dispositif représenté sur la figure 1, le dispositif ici schématisé comporte en outre au moins un épurateur, 18, 19, 20, 21 placé en amont d'au moins un mélangeur 7 ou 8. Par conséquent au moins l'un des gaz purs à mélanger pourra être épuré avant d'entrer dans le mélangeur. L'ajout d'un épurateur en amont du mélangeur permet de retirer des impuretés spécifiques d'au moins un des gaz purs G1 à G4 en entrée que l'on ne pourrait pas retirer du mélange final en raison de contraintes techniques et/ou de sécurité. Selon, les objectifs à atteindre, un seul gaz d'entrée sera épuré en amont d'au moins un mélangeur 7 et 8, ou encore plusieurs gaz d'entrée seront épurés avant d'être mélangés. Enfin, selon un mode de réalisation de l'invention, tous les gaz à mélanger seront en premier lieu épurés.

De manière générale, la solution proposée par la présente invention est donc constituée d'une part d'un mélangeur permettant de générer sur site les gaz d'instrumentation et d'autre part d'un épurateur placé en aval du mélangeur et en amont de l'analyseur. Ces deux éléments sont associés et constituent un seul équipement.

Le mélangeur permet d'assurer la stabilité de la composition du mélange dans le temps. Il peut être composé soit de régulateurs de débits massiques soit d'orifices soniques selon la dynamique de débit souhaitée par le client. L'épurateur permet d'éliminer les impuretés critiques que sont par exemple les hydrocarbures en les convertissant en CO₂ et H₂O, par exemple selon un procédé catalytique. Cet épurateur pouvant également comprendre un adsorbant en lits successifs permettant de piéger ces impuretés. Par exemple un premier lit de tamis pour piéger H₂O et un second lit de zéolithe pour piéger le CO₂.

Les composants du mélangeur permettent d'obtenir une précision de réalisation du mélange inférieure ou égale à 0.5 % absolus sur la concentration du composant minoritaire. La production sur site client permet de s'affranchir des potentielles fluctuations des concentrations des mélanges formant les gaz d'instrumentation. Par exemple, dans le cas d'un FID, la production sur site client permet de s'affranchir des potentielles fluctuations de la concentration en hydrogène dans le mélange H₂/He ou de celle en oxygène dans le mélange O₂/N₂. La garantie de cette précision permet au client d'améliorer la reproductibilité de ses analyses.

L'épurateur permet d'obtenir en amont de l'analyseur un niveau d'impuretés hydrocarbonées inférieures à celle perturbant son analyse. Son fonctionnement continu permet de maintenir le « bruit de fond » de l'analyseur à des niveaux satisfaisants pour l'analyse de basses teneurs en hydrocarbures. Pour les FID les impuretés critiques sont les hydrocarbures, pour l'analyse de 100 ppbv d'hydrocarbures, l'épurateur devra permettre d'atteindre des teneurs inférieures à 10 pbbv en hydrocarbures.

L'équipement complet permet de garantir au client une stabilité de la composition du mélange dans le temps, de par la précision du mélangeur, et une pureté constante délivrée au point d'utilisation, de par les performances de l'épurateur. Cette solution présente donc l'avantage de fournir au client un mélange dont les caractéristiques essentielles sont stables au cours du temps. Ainsi la solution proposée permet de réduire la contribution des gaz d'alimentation des analyseurs à l'incertitude des analyses. En outre le fait d'assembler le mélangeur et l'épurateur permet au client une facilité d'utilisation et une rapidité de mise en oeuvre avantageuses par rapport à une distribution en bouteilles.

Il doit être évident que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Dispositif (1) d'analyse d'un fluide (2) comprenant :
- un analyseur (3) ; et
- un système (4) d'alimentation en gaz d'instrumentation (5, 6) dudit analyseur (3) ;
**caractérisé en ce que** le système (4) d'alimentation comprend au moins un mélangeur (7, 8) générant ledit gaz d'instrumentation (5, 6) et au moins un épurateur (9, 10) situé en aval dudit au moins un mélangeur (7, 8) et en amont de l'analyseur (3).

2. Dispositif selon la revendication 1, dans lequel le système (4) d'alimentation en gaz d'instrumentation (5, 6) dudit analyseur (3) comprend en outre au moins un épurateur (18, 19, 20, 21) situé en amont d'au moins un mélangeur (7, 8).

3. Dispositif selon l'une des revendications 1 ou 2, dans lequel l'analyseur (3) est un détecteur à ionisation de flamme.

4. Dispositif selon l'une des revendications 1 à 3, dans lequel le système d'alimentation (4) alimente l'analyseur (3) par deux mélanges de gaz d'instrumentation, l'un (5) étant un mélange Hydrogène/Hélium, l'autre (6) un mélange Oxygène/Azote ; le système d'alimentation (4) étant constitué d'un premier épurateur (9) situé en aval d'un premier mélangeur (7) générant le mélange (5) Hydrogène/Hélium et en amont de l'analyseur (3) et d'un deuxième épurateur (10) situé en aval d'un deuxième mélangeur (8) générant le mélange (6) Oxygène/Azote et en amont de l'analyseur (3).

5. Dispositif selon l'une quelconque des revendications 1 à 4 **caractérisé en ce que** ledit au moins un mélangeur (7, 8) comprend au moins un composant choisi parmi un régulateur de débits massiques, des orifices soniques, des barillets d'orifices calibrés de différents diamètres ou des vannes de régulation.

6. Dispositif selon l'une quelconque des revendications 1 à 5 **caractérisé en ce que** ledit au moins un épurateur (9, 10) comporte un ou plusieurs des éléments choisis parmi les éléments suivants : un filtre à particules, un catalyseur comprenant des métaux nobles et/ou des oxydes métalliques et un piège cryogénique.

7. Dispositif selon l'une quelconque des revendications 1 à 6, dans lequel ledit au moins un épurateur (9, 10) comporte également au moins un adsorbant.

8. Dispositif selon l'une quelconque des revendications 1 à 7, dans lequel ledit au moins un épurateur (9, 10) comporte également un moyen d'avertissement de sa fin de durée de vie garantie.

9. Dispositif selon l'une quelconque des revendications 1 à 8 **caractérisé en ce qu'**un régulateur de pression est situé en aval dudit au moins un épurateur (9, 10).

10. Système (4) d'alimentation en gaz d'instrumentation (5, 6) d'un analyseur (3) de fluide (2), constitué d'au moins un mélangeur (7, 8), générant ledit gaz d'instrumentation (5, 6), associé à au moins un épurateur (9, 10) situé en aval dudit au moins un mélangeur (7, 8), **caractérisé en ce qu'**il est intégré dans un même dispositif (1), tel que défini à l'une des revendications 1 à 9.

11. Système (4) d'alimentation en gaz d'instrumentation (5, 6) d'un analyseur (3) de fluide (2) selon la revendication 10, comportant en outre au moins un épurateur (18, 19, 20, 21) situé en amont d'au moins un mélangeur (7, 8).

12. Système (4) d'alimentation en gaz d'instrumentation (5, 6) d'un détecteur à ionisation de flamme selon l'une des revendications 10 ou 11, constitué de deux mélangeurs (7, 8) générant chacun un mélange de gaz d'instrumentation (5, 6), chaque mélangeur étant situé en amont d'un épurateur (9, 10).

13. Système (4) d'alimentation en gaz d'instrumentation (5, 6) selon la revendication 12 **caractérisé en ce qu'**un épurateur (18, 19, 20, 21) par gaz d'entrée (G1 à G4) est placé en amont de chaque mélangeur (7, 8).

14. Procédé de délivrance d'au moins un mélange de gaz (5, 6) vers un analyseur (3) de fluide (2) comprenant les étapes :
a) mélange d'au moins deux gaz purs mettant en oeuvre un mélangeur (7, 8),
b) épuration du mélange obtenu à l'étape a) au moyen d'un épurateur (9, 10) générant un mélange de gaz d'instrumentation pour ledit analyseur (3) ; **caractérisé en ce que** ces étapes se déroulent sur un même site au moyen d'un unique système (4) d'alimentation en gaz d'instrumentation (5, 6) tel que défini à l'une des revendications 10 à 13.

15. Procédé de délivrance d'au moins un mélange de gaz (5, 6) vers un analyseur (3) de fluide (2) selon la revendication 14 comprenant en outre une étape d'épuration d'au moins un gaz à mélanger, antérieure à l'étape a).

## Claims

1. Device (1) for analyzing a fluid (2), comprising:
- an analyzer (3); and
- a feed system (4) for supplying said analyzer with instrumentation gases (5, 6),
**characterized in that** the feed system (4) comprises at least one mixer (7, 8) that generates said instrumentation gas (5, 6) and at least one purifier (9, 10) located downstream of said at least one mixer (7, 8) and upstream of the analyzer (3).

2. Device according to Claim 1, in which the system (4) for supplying said analyzer (3) with instrumentation gases (5, 6) further includes at least one purifier (18, 19, 20, 21) located upstream of at least one mixer (7, 8).

3. Device according to either of Claims 1 and 2, in which the analyzer (3) is a flame ionization detector.

4. Device according to one of Claims 1 to 3, in which the feed system (4) supplies the analyzer (3) with two instrumentation gas mixtures, one (5) being a hydrogen/helium mixture and the other (6) an oxygen/nitrogen mixture, the feed system (4) being formed by a first purifier (9) located downstream of a first mixer (7) that generates the hydrogen/helium mixture (5) and upstream of the analyzer (3) and by a second purifier (10) located downstream of a second mixer (8) that generates the oxygen/nitrogen mixture (6) and upstream of the analyzer (3).

5. Device according to any one of Claims 1 to 4, **characterized in that** said at least one mixer (7, 8) comprises at least one component chosen from a mass flow rate regulator, sonic orifices, sets of calibrated orifices of different diameters, or regulating valves.

6. Device according to any one of Claims 1 to 5, **characterized in that** said at least one purifier (9, 10) comprises one or more elements chosen from the following elements: a particulate filter, a catalyst comprising noble metals and/or metal oxides, and a cryogenic trap.

7. Device according to any one of Claims 1 to 6, in which said at least one purifier (9, 10) also includes at least one adsorbent.

8. Device according to any one of Claims 1 to 7, in which said at least one purifier (9, 10) also includes a means of warning that it is coming to the end of its guaranteed lifetime.

9. The device according to any one of Claims 1 to 8, **characterized in that** a pressure regulator is located downstream of said at least one purifier (9, 10).

10. Feed system (4) for supplying a fluid (2) analyzer (3) with instrumentation gases (5, 6), consisting of at least one mixer (7, 8), which generates said instrumentation gas (5, 6), combined with at least one purifier (9, 10) located downstream of said at least one mixer (7, 8), **characterized in that** it is integrated into one and the same device (1), as defined in one of Claims 1 to 9.

11. Feed system (4) for supplying a fluid (2) analyzer (3) with instrumentation gases (5, 6) according to Claim 10, which further includes at least one purifier (18, 19, 20, 21) located upstream of at least one mixer (7,8).

12. Feed system (4) for supplying a flame ionization detector with instrumentation gases (5, 6) according to either of Claims 10 and 11, formed by two mixers (7, 8) each generating an instrumentation gas mixture (5, 6), each mixer being located upstream of a purifier (9, 10).

13. Feed system (4) for supplying an instrumentation gas (5, 6) according to Claim 12, **characterized in that** one purifier (18, 19, 20, 21) per entry gas (G1 to G4) is placed upstream of each mixer (7, 8).

14. Method of delivering at least one gas mixture (5, 6) to a fluid (2) analyzer (3), comprising the following steps:
a) a mixture of at least two pure gases is formed using a mixer (7, 8); and
b) the mixture obtained in step a) is purified by means of a purifier (9, 10) that generates an instrumentation gas mixture for said analyzer (3), **characterized in that** these steps are carried out on one and the same site by means of a single feed system (4) for supplying an instrumentation gas (5, 6) as defined in one of Claims 10 to 13.

15. Method of delivering at least one gas mixture (5, 6) to a fluid (2) analyzer (3) according to Claim 14, which further includes, prior to step a), a step of purifying at least one gas to be mixed.

## Patentansprüche

1. Vorrichtung (1) für die Analyse eines Fluids (2), mit:
- einem Analysator (3); und
- einem System (4) für die Versorgung mit Messgas (5, 6) des Analysators (3);
**dadurch gekennzeichnet, dass** das Versorgungssystem (4) wenigstens einen Mischer (7, 8), der das Messgas (5, 6) erzeugt, und wenigstens einen Abscheider (9, 10), der sich stromabseitig von dem wenigstens einen Mischer (7, 8) und stromaufseitig von dem Analysator (3) befindet, enthält.

2. Vorrichtung nach Anspruch 1, wobei das System (4) für die Versorgung mit Messgas (5, 6) des Analysators (3) außerdem wenigstens einen Abscheider (18, 19, 20, 21) aufweist, der sich stromaufseitig von wenigstens einem Mischer (7, 8) befindet.

3. Vorrichtung nach einem der Ansprüche 1 oder 2, wobei der Analysator (3) ein Flammenionisationsdetektor ist.

4. Vorrichtung nach einem der Ansprüche 1 bis 3, wobei das Versorgungssystem (4) den Analysator (3) mit zwei Messgasgemischen versorgt, wovon eines (5) ein Wasserstoff/Helium-Gemisch ist und das andere (6) ein Sauerstoff/Stickstoff-Gemisch ist; wobei das Versorgungssystem (4) aus einem ersten Abscheider (9), der sich stromabseitig von einem ersten Mischer (7) befindet, der das Wasserstoff/Helium-Gemisch (5) erzeugt, und stromaufseitig von dem Analysator (3) befindet, und aus einem zweiten Abscheider (10), der sich stromabseitig von einem zweiten Mischer (8), der das Sauerstoff/Stickstoff-Gemisch (6) erzeugt, und stromaufseitig von dem Analysator (3) befindet, gebildet ist.

5. Vorrichtung nach einem der Ansprüche 1 bis 4, **dadurch gekennzeichnet, dass** der wenigstens eine Mischer (7, 8) wenigstens eine Komponente enthält, die aus einem Massendurchsatzregulierer, Schallöffnungen, Trommeln mit kalibrierten Öffnungen mit unterschiedlichen Durchmessern oder Regulierungsventilen gewählt ist.

6. Vorrichtung nach einem der Ansprüche 1 bis 5, **dadurch gekennzeichnet, dass** der wenigstens eine Abscheider (9, 10) ein oder mehrere Elemente enthält, die aus den folgenden Elementen gewählt sind: einem Partikelfilter, einem Katalysator, der Edelmetalle und/oder Metalloxide aufweist, und einer Kühlfalle.

7. Vorrichtung nach einem der Ansprüche 1 bis 6, wobei der wenigstens eine Abscheider (9, 10) außerdem wenigstens ein Adsorptionsmittel enthält.

8. Vorrichtung nach einem der Ansprüche 1 bis 7, wobei der wenigstens eine Abscheider (9, 10) außerdem ein Warnmittel für sein garantiertes Lebensdauerende enthält.

9. Vorrichtung nach einem der Ansprüche 1 bis 8, **dadurch gekennzeichnet, dass** sich stromabseitig von dem wenigstens einen Abscheider (9, 10) ein Druckregulierer befindet.

10. System (4) für die Versorgung mit Messgas (5, 6) eines Analysators (3) für Fluid (2), das aus wenigstens einem Mischer (7, 8) gebildet ist, der das Messgas (5, 6) erzeugt, und wenigstens einem Abscheider (9, 10) zugeordnet ist, der sich stromabseitig von dem wenigstens einen Mischer (7, 8) befindet, **dadurch gekennzeichnet, dass** es in dieselbe Vorrichtung (1) wie in einem der Ansprüche 1 bis 9 definiert integriert ist.

11. System (4) für die Versorgung mit Messgas (5, 6) eines Analysators (3) für Fluid (2) nach Anspruch 10, das außerdem wenigstens einen Abscheider (18, 19, 20, 21) enthält, der sich stromaufseitig von wenigstens einem Mischer (7, 8) befindet.

12. System (4) für die Versorgung mit Messgas (5, 6) eines Flammenionisationsdetektors nach einem der Ansprüche 10 oder 11, das aus zwei Mischern (7, 8) gebildet ist, die jeweils ein Messgasgemisch (5, 6) erzeugen, wobei sich jeder Mischer stromaufseitig von einem Abscheider (9, 10) befindet.

13. System (4) für die Versorgung mit Messgas (5, 6) nach Anspruch 12, **dadurch gekennzeichnet, dass** ein Abscheider (18, 19, 20, 21) pro Gaseinlass (G1 bis G4) stromaufseitig von jedem Mischer (7, 8) angeordnet ist.

14. Verfahren zum Ausgeben wenigstens eines Gasgemisches (5, 6) zu einem Analysator (3) für Fluid (2), das die folgenden Schritte enthält:
a) Mischen wenigstens zweier reiner Gase unter Verwendung eines Mischers (7, 8),
b) Abscheiden des im Schritt a) erhaltenen Gemisches mittels eines Abscheiders (9, 10), der ein Messgasgemisch für den Analysator (3) erzeugt;
**dadurch gekennzeichnet, dass** diese Schritte an derselben Stelle mittels eines einzigen Systems (4) für die Versorgung mit Messgas (5, 6) wie in einem der Ansprüche 10 bis 13 definiert ausgeführt werden.

15. Verfahren zum Ausgeben wenigstens eines Gasgemisches (5, 6) zu einem Analysator (3) für Fluid (2) nach Anspruch 14, das außerdem einen Schritt des Abscheidens wenigstens eines zu mischenden Gases vor dem Schritt a) enthält.
